# FASCICULE DE BREVET EUROPEEN

(11) **EP 3 075 411 B1**
(45) Date de publication et mention de la délivrance du brevet: **25.10.2017**
(21) Numéro de dépôt: 16162484.6
(22) Date de dépôt: 25.03.2016
(51) Int. Cl.: A61N 1/05, A61N 1/36

(54) **SONDE MULTIÉLECTRODE À COMMANDE MULTIPLEXÉE, NOTAMMENT POUR STIMULATION CARDIAQUE, ET PROCÉDÉ DE CONNEXION ASSOCIÉ**
MULTIELEKTRODENSONDE MIT MULTIPLEX-STEUERUNG, INSBESONDERE ZUR HERZSTIMULATION, UND ENTSPRECHENDES ANSCHLUSSVERFAHREN
MULTI-ELECTRODE PROBE WITH MULTIPLEXED CONTROL, IN PARTICULAR FOR CARDIAC STIMULATION, AND ASSOCIATED CONNECTION METHOD

(30) Priorité: 03.04.2015 FR 1552894
(43) Date de publication de la demande: 05.10.2016
(73) Titulaire: Sorin CRM SAS, 92140 Clamart Cedex (FR)
(72) Inventeur: SHAN, Nicolas, 91260 Juvisy sur Orge (FR); D'HIVER, Philippe, 92320 Chatillon (FR)
(74) Mandataire: Grünecker Patent- und Rechtsanwälte PartG mbB

(56) Documents cités:
- WO-A1-2009/018426
- WO-A1-2012/087370
- WO-A2-2004/052182
- US-A1- 2003 149 456

## Description

L'invention concerne les "dispositifs médicaux implantables actifs" tels que définis par la directive 90/385/CEE du 20 juin 1990 du Conseil des communautés européennes, et plus précisément les implants dits "multisite" permettant de recueillir des potentiels électriques et/ou délivrer des impulsions électriques de stimulation de façon sélective en un ou plusieurs sites d'un ensemble de sites, notamment dans des applications de cardiologie et de neuromodulation.

Le développement récent de tels appareils de stimulation multisite a conduit à la multiplication du nombre d'électrodes, de manière à permettre le choix d'un ou plusieurs sites de détection/stimulation optimisant le fonctionnement de l'appareil.

Dans la suite, on fera principalement référence à des électrodes utilisées pour l'application d'impulsions de stimulation, mais cette fonction n'est pas limitative et l'invention s'applique aussi bien au cas d'électrodes utilisées à des fins de détection de potentiels électriques recueillis en des sites précis, une même électrode pouvant d'ailleurs être utilisée à la fois pour la détection et la stimulation.

Dans le cas particulier des dispositifs cardiaques implantés de resynchronisation ventriculaire dits "CRT" (*Cardiac Resynchronization Therapy*), que l'on cite ici à titre d'exemple non limitatif, on implante dans le patient un appareil muni d'électrodes permettant de stimuler l'un et l'autre des ventricules. La stimulation du ventricule droit (et de l'oreillette droite) est opérée par une sonde endocavitaire classique, mais pour le ventricule gauche, l'accès étant plus complexe, la stimulation est généralement opérée au moyen d'une sonde introduite dans le sinus coronaire du ventricule droit puis poussée dans une veine coronaire sur l'épicarde, de façon que l'extrémité de la sonde vienne se placer face au ventricule gauche. Cette procédure est toutefois assez délicate, car le diamètre des vaisseaux coronaires se réduit avec l'avancement de la sonde, de sorte qu'il n'est pas toujours facile de trouver la position optimale lors de l'implantation. La proximité du nerf phrénique peut par ailleurs conduire à des stimulations inappropriées.

Pour pallier ces difficultés, on a développé des sondes dites "multiélectrode", pourvues par exemple de huit électrodes ou plus, et il est possible de choisir après implantation l'électrode qui correspond au site sur lequel la stimulation est la plus efficace. Cette sélection de l'électrode peut être réalisée de façon automatique par une mesure des pics d'accélération endocardiaque (PEA), par une mesure de bioimpédance, ou à partir tout autre capteur permettant de donner une information représentative de l'état hémodynamique du patient. Elle peut également être opérée manuellement par le praticien, au moyen d'un programmateur approprié commandant le générateur.

Ces sondes de stimulation doivent présenter un diamètre le plus petit possible afin d'étendre les possibilités d'implantation tout en étant le moins traumatique possible pour l'organisme.

D'autre part, la multiplication du nombre d'électrodes engendre des problématiques de connexion délicates au niveau de la liaison avec le générateur d'impulsions de stimulation.

Les difficultés rencontrées sont du même ordre dans les applications de neuromodulation opérant par stimulation multipoint du système nerveux central. La neuromodulation consiste par exemple à implanter une microsonde dans le réseau veineux cérébral de manière à atteindre des zones cibles bien spécifiques du cerveau afin d'y appliquer des impulsions électriques de stimulation pour traiter certaines pathologies telles que la maladie de Parkinson, l'épilepsie, etc. Il peut également s'agir de stimuler le système nerveux périphérique, les électrodes étant alors placées au niveau des nerfs ou des muscles.

Avec les techniques actuellement connues, l'augmentation du nombre d'électrodes à connecter a un fort impact sur l'encombrement et le coût de l'électronique interne du boitier, de la tête du boitier et de la sonde, de sorte que l'on préfère utiliser un circuit démultiplexeur qui permet de ne décoder des signaux et tensions que sur un nombre limité de conducteurs (typiquement 2 ou 4). Il serait toutefois dans le principe intéressant de pouvoir augmenter le nombre d'électrodes tout en réduisant globalement le volume de connexion entre la source de signaux et la sonde, en ne nécessitant que ces 2 ou 4 conducteurs pour alimenter et commander le démultiplexeur.

Cette technique de multiplexage/démultiplexage est déjà mise en oeuvre dans des applications de cardiologie et neuromodulation.

Dans certains cas, le circuit de démultiplexage se situe dans la partie distale du dispositif, à proximité des électrodes, en étant incorporé dans le corps de sonde. Les EP 1 938 861 A1, EP 2 465 425 A1 et US 2011/301665 A1 décrivent de tels agencements. Mais on notera que dans ces structures connues, les dimensions transversales du corps de sonde dans sa partie distale sont plus grandes du fait de la nécessité d'intégrer de façon étanche le circuit électronique de démultiplexage. Alternativement, pour éviter cette difficulté, un module intermédiaire de dimensions assez grandes est prévu à mi-chemin entre le générateur et l'extrémité de sonde (voir notamment le EP 2 727 623 A1), ce qui complique la pose et crée un risque supplémentaire du fait de la nécessité d'implanter un élément additionnel.

Par ailleurs, l'évolution des structures de conducteurs et des technologies d'électrodes de sondes est telle qu'il devient possible aujourd'hui de réaliser des sondes de très petites dimensions permettant de stimuler et de détecter les événements électriques du coeur.

De telles structures peuvent utiliser des conducteurs d'un diamètre de 40 à 60 µm et peuvent donc comporter une multitude de conducteurs isolés les uns des autres typiquement, jusqu'à 100 conducteurs distincts dans un diamètre inférieur à 0,5 mm - et l'on ne sait pas associer de telles structures à des multiplexeurs sans immédiatement rencontrer les problèmes précités, notamment en termes d'encombrement, de complexité et de coût.

Le WO 2012/087370 A1 (correspondant au US 8 639 341 B2) décrit un agencement où le circuit de démultiplexage est logé dans une région du corps du connecteur, avec un bloc de support et de connexion intercalé entre les conducteurs multiplexés et les conducteurs non multiplexés. Ce bloc comprend au centre un support allongé recevant le circuit intégré de démultiplexage, circuit qui est électriquement relié aux deux groupes de conducteurs respectifs arrivant de part et d'autre du support. Cet agencement, s'il permet d'incorporer au connecteur les moyens de démultiplexage, présente cependant l'inconvénient d'un encombrement important qui augmente de façon significative la longueur du connecteur.

La présente invention vise à pallier les limitations de l'état de la technique et à proposer une solution de connexion entre un circuit démultiplexeur et une sonde multiélectrode qui soit fiable et protégée tout en étant de faible encombrement, et qui puisse notamment se loger au voisinage du connecteur de la sonde, ne pas nécessiter d'accroissement de diamètre de cette dernière, et simplifier la structure de la connectique du boitier associé.

L'invention propose à cet effet, selon un premier aspect, une sonde multiélectrode comprenant, de manière en elle-même connue d'après le WO 2012/087370 A1 précité :
- un connecteur pour une liaison de commande et/ou d'alimentation et/ou de transfert de données ;
- un circuit intégré de démultiplexage apte à recevoir en entrée sur des premiers conducteurs des signaux électriques de commande et/ou d'alimentation et/ou de transfert de données en provenance de la liaison de commande et/ou d'alimentation et/ou de transfert de données et relié en sortie à une pluralité de seconds conducteurs contenus dans la sonde ;
- un élément porte-circuit et de connexion possédant un corps formant support pour le circuit intégré de démultiplexage et logé dans un corps de connecteur portant le connecteur ; et
- un ensemble d'électrodes s'étendant le long de la sonde et reliées aux seconds conducteurs. Selon l'invention, l'élément porte-circuit et de connexion :
- est de forme généralement cylindrique et reçoit le circuit intégré sur l'une de ses faces d'extrémité ;
- définit un ensemble de cavités de connexion présentant la forme de gorges s'étendant axialement à la périphérie du corps de l'élément, avec au moins les seconds conducteurs répartis à la périphérie du corps autour d'un axe général dudit corps et disposés dans lesdites cavités de connexion ; et
- comprend un ensemble d'éléments de connexion noyés dans le matériau du corps, émergeant au niveau d'une région de l'élément supportant le circuit intégré et au niveau de cavités respectives, ces éléments de connexion reliant électriquement lesdits premiers et/ou seconds conducteurs à des bornes respectives dudit circuit intégré.

Selon diverses caractéristiques subsidiaires avantageuses :
- les éléments conducteurs possèdent chacun une partie émergente s'étendant dans une telle gorge respective sur au moins une partie de sa longueur axiale ;
- les éléments conducteurs possèdent chacun une première partie noyée s'étendant généralement axialement et dont une extrémité débouche au niveau d'une surface adjacente au circuit intégré ;
- chaque élément conducteur comprend une partie intermédiaire noyée s'étendant généralement radialement entre la première partie noyée et la partie émergente s'étendant dans la gorge ;
- le circuit intégré est protégé par un capot fixé de façon hermétique sur le corps de l'élément porte-circuit et de connexion ;
- l'élément porte-circuit et de connexion est réalisé par une technologie céramique-métal, le corps de l'élément étant constitué par une région en céramique et les éléments de connexion étant constituées par des régions en métal ;
- l'élément porte-circuit et de connexion comporte en outre une région métallique pour le soudage du capot ;
- cette région métallique est une région annulaire entourant le circuit intégré ;
- le capot est également réalisé en technologie céramique-métal et comprend une région métallique homologue de la région métallique entourant le circuit intégré ;
- les conducteurs sont entourés à leur extrémité libre reçue dans sa cavité respective par un manchon métallique ;
- la sonde comprend en outre un élément de transition apte à retenir les seconds conducteurs dans une configuration correspondant à l'agencement des cavités pour lesdits seconds conducteurs ;
- la sonde comprend en outre un élément de transition apte à retenir les premiers conducteurs dans une configuration correspondant à l'agencement des cavités pour lesdits premiers conducteurs ;
- le corps de l'élément porte-circuit et de connexion comprend au moins deux régions cylindriques généralement coaxiales et de diamètres différents, chaque région possédant une pluralité de gorges s'étendant axialement à sa périphérie.

Selon un deuxième aspect, l'invention propose un procédé de connexion de conducteurs à un circuit de démultiplexage dans une sonde multiélectrode de stimulation localisée du type exposé ci-dessus, ce procédé comprenant des étapes consistant à :
a) monter le circuit intégré de démultiplexage sur l'une des faces d'extrémité de l'élément ;
b) relier des bornes du circuit aux parties émergentes des régions de connexion voisines du circuit ; et
c) relier les conducteurs aux parties émergentes des régions de connexion au niveau desdites cavités.

Selon diverses caractéristiques subsidiaires avantageuses :
- l'étape b) est mise en oeuvre à l'aide de fils de liaison ;
- les étapes a) et b) sont mises en oeuvre simultanément par une technique de montage de puce retournée ;
- l'étape c) est réalisé par tirs laser ;
- les conducteurs sont entourés à leur extrémité libre reçue dans sa cavité respective par un manchon métallique ;
- le matériau métallique du manchon est le même que le matériau métallique de l'élément porte-circuit et de connexion ;
- le procédé comprend également des étapes consistant à :
   d) prévoir un capot au moins en partie métallique pour la protection du circuit intégré ;
   e) prévoir sur l'élément porte-circuit et de connexion une région métallique entourant le circuit intégré ; et
   f) fixer le capot sur ledit élément par tirs laser au niveau de ladite région métallique.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 est une vue d'ensemble en élévation de côté d'une sonde multiélectrode à commande multiplexée selon l'invention.
La Figure 2 est une vue en perspective d'un module porte-circuit et de connexion de la sonde de la Figure 1.
La Figure 3 et une vue en perspective du module recevant un certain nombre de conducteurs pour électrodes et un capot de protection du circuit monté sur le module.
La Figure 4 est une vue en élévation de côté du module de la Figure 2.
La Figure 5 est une vue en élévation de face du module des Figures 2 et 4.
La Figure 6 est une vue en coupe axiale d'un module ayant une géométrie différence de celle des Figures 2 à 5, équipé du circuit, du capot, des conducteurs et d'une enveloppe de protection.
La Figure 7 est une vue de face de la représentation de la Figure 6.
Les Figures 8 et 9 illustrent l'assemblage du module des Figures 6 et 7 avec son capot.
La Figure 10 illustre en perspective un aménagement d'un conducteur destiné à être raccordé au module.
La Figure 11 est une vue en coupe transversale partielle et à échelle agrandie de la région de connexion entre un tel conducteur et le module.
La Figure 12 est une vue en perspective éclatée d'un module doté de son capot, de conducteurs et de pièces de transition pour les conducteurs.
La Figure 13 est une vue en perspective d'une variante de l'une des deux pièces de transition.
La Figure 14 est une vue en coupe axiale d'une variante de réalisation du module selon l'invention équipé du circuit, du capot, et des conducteurs.
La Figure 15 est une vue de dos du module équipé de la Figure 14.
La Figure 16 illustre de façon schématique les étapes successives de montage d'une sonde multiélectrode réalisée selon les enseignements de l'invention.

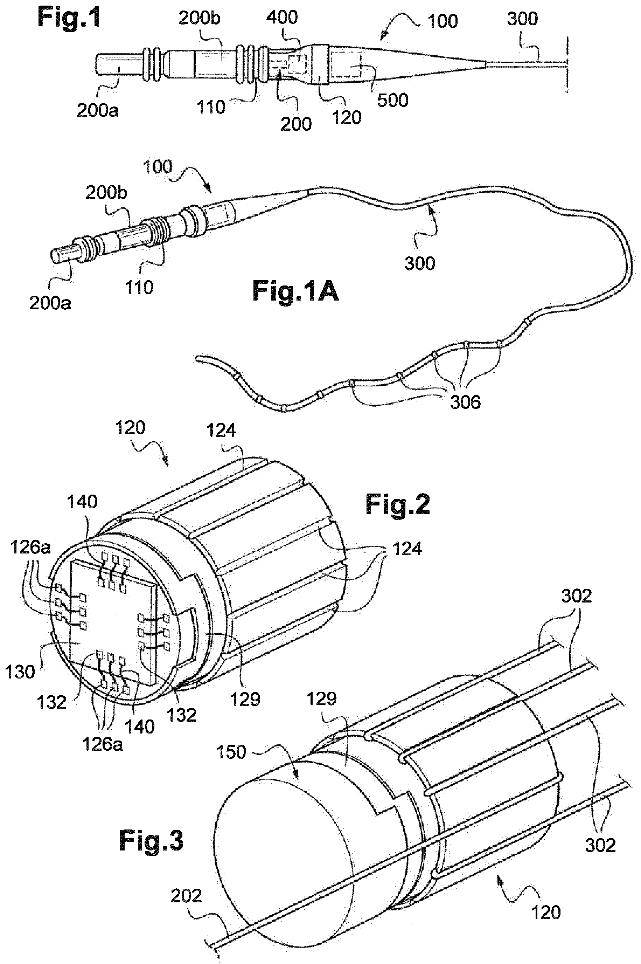

En référence tout d'abord aux Figures 1 et 1A, on a représenté une sonde multiélectrode à démultiplexage intégré qui comprend : un corps intermédiaire effilé 100 abritant un module porte-circuit et de connexion 120 tel qu'on va le décrire dans la suite ; un connecteur 110 avec une liaison de commande et/ou d'alimentation et/ou de transfert de données 200, typiquement à deux ou quatre conducteurs ; et la sonde proprement dite 300, abritant une pluralité de conducteurs 302, typiquement de huit paires de conducteurs à plusieurs dizaines de paires de conducteurs, ces conducteurs étant reliés à une série d'électrodes de stimulation 306 espacées le long de la sonde.

Dans l'exemple illustré Figures 1 et 1A la liaison 200 à 2 (ou 4) conducteurs est une liaison incorporée au connecteur 110, et reliée aux 2 (ou 4) pôles 200a, 200b de ce connecteur. Il s'agit là d'un mode de réalisation particulièrement avantageux, puisqu'il permet d'inclure l'étage multiplexeur au sein même du connecteur, donc sans surcroit d'encombrement, notamment en dimension diamétrale.

Mais on gardera à l'esprit que l'invention peut être appliquée à de nombreuses autres configurations, chaque fois qu'il est nécessaire d'interfacer à un dispositif une sonde multiélectrode de détection/stimulation au moyen d'un étage multiplexeur/démultiplexeur. Le dispositif en question peut en particulier être tout type de neurostimulateur ou stimulateur cardiaque, et notamment se présenter sous forme d'un dispositif autonome muni de sa propre alimentation électrique (capsule implantable dite *leadless*) et prolongé par une sonde multiélectrode auquel il est relié par un système de communication, etc.

De façon avantageuse mais non limitative, le connecteur 110 est dans l'exemple illustré un connecteur standard de type *IS-1* (auquel sont reliés deux conducteurs d'amenée 202 logés dans le corps 100, suivant l'exemple illustré) ou *IS-4* (quatre conducteurs). La sonde proprement dite 300 présente dans le présent exemple dix conducteurs 302.

En référence maintenant aux Figures 2 à 5, on a illustré un module 120 logé à l'intérieur du corps 100, dont les fonctions principales sont de recevoir un circuit électronique de démultiplexage répondant aux signaux arrivant par les conducteurs d'amenée 202, reliés à la liaison 200 par le connecteur 110, pour sélectivement appliquer des impulsions de stimulation à l'une des électrodes 306 (ou à un sous-ensemble donné desdites électrodes).

Ce module comprend un corps principal généralement cylindrique délimité par une paroi extérieure cylindrique 122 dans laquelle sont formées une série de gorges longitudinales 124 destinées comme on le verra dans la suite à réaliser une connexion avec les conducteurs de la liaison 200 et les conducteurs de la partie de sonde 300. Dans le présent exemple de réalisation, ces gorges 124 sont au nombre de douze : deux pour les conducteurs 202, qui partent en direction de la liaison 200, et dix pour les conducteurs 302, qui partent en direction de la partie de sonde 300.

Sur l'une de ses faces d'extrémités, ici sur sa face proximale, le module 120 reçoit un circuit intégré 130 destiné à assurer la fonction de démultiplexage des signaux reçus via la liaison 200 pour commander les impulsions comme décrit ci-dessus.

Ce circuit intégré comporte en surface (ou, en variante et de façon non illustrée, sur ses flancs), des plots ou zones conductrices 132 permettant la connexion du circuit intégré à son environnement par des fils de liaison (fils de *bonding*) 140, de façon connue en soi. Dans le corps du module sont noyés un ensemble d'éléments conducteurs 126 destinés à assurer la connexion entre le circuit 130 et les conducteurs de la liaison 200 et de la partie de sonde 300, comme on le décrira en détail dans la suite.

La Figure 3 illustre un capot 150 destiné à être fixé de façon hermétique sur la face du module 120 recevant le circuit 130, comme on le décrira dans la suite, de manière à protéger le circuit 130 et les fils de *bonding.*

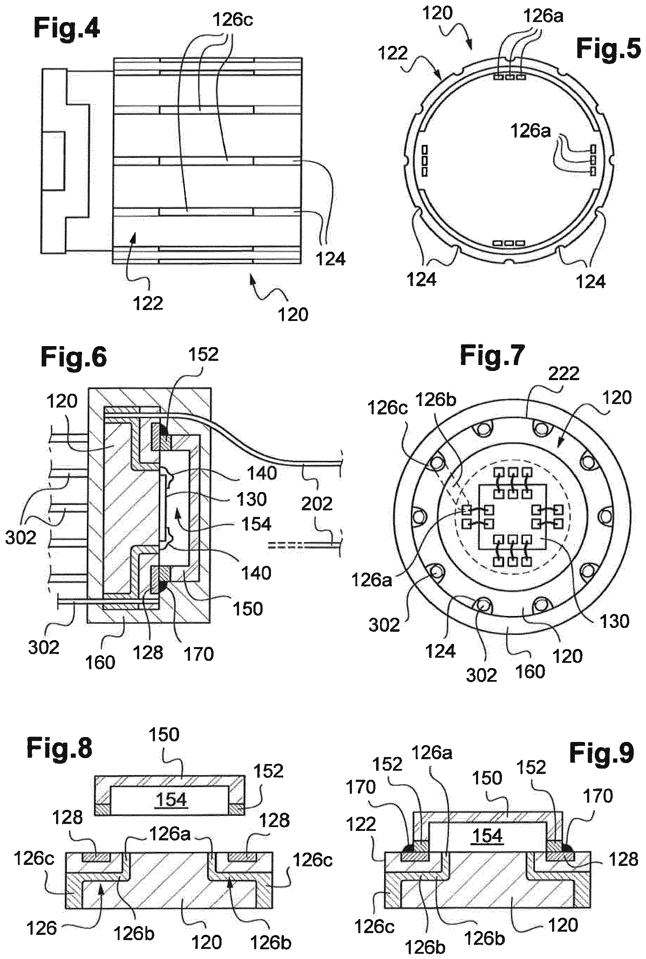

En référence aux Figures 6 à 9, qui illustrent un module ayant une géométrie légèrement différente de celle des Figures 2 à 5 (essentiellement avec une longueur axiale plus courte), on peut observer la configuration des éléments conducteurs internes 126 du module 120. Chaque élément comprend une première partie 126a s'étendant axialement et affleurant au niveau de son extrémité libre sur la face du corps du module 120 qui porte le circuit 130, en débouchant à la périphérie dudit circuit. Cette première partie se prolonge par une partie 126b orientée généralement radialement, en direction de l'une des gorges longitudinales 124 destinées chacune à recevoir un conducteur 202 ou 303. Cette partie 126b se termine par une partie 126c qui débouche dans la gorge 124 en s'étendant sur au moins une partie de son étendue axiale.

La liaison électrique entre le circuit de démultiplexage 130 et chacun des conducteurs s'effectue, côté circuit 130, par les fils de *bonding* 140 soudés d'une part sur les plots conducteurs 132 du circuit et d'autre part sur les régions affleurantes des parties 126a des éléments conducteurs noyés, respectivement. Côté conducteurs 202, 302, cette liaison s'effectue comme on va le voir en détail dans la suite par mise en contact de parties dénudées de ces conducteurs avec les parties 126c des éléments conducteurs qui débouchent dans les gorges respectives 124.

Selon une variante de réalisation non illustrée, la connexion électrique entre les plots conducteurs du circuit 130 et les éléments conducteurs respectifs 126 peut être réalisée selon la technologie connue dite de "puce retournée" (*flip-chip*), la puce disposant de contacts légèrement saillants sur sa face tournée vers le module 120 et ces contacts venant se connecter aux zones affleurantes des parties 126a des éléments conducteurs noyés 126. Cette technique permet d'éviter le recours à des fils de *bonding.*

Avantageusement, le corps du module 120 et ses éléments conducteurs 126 sont réalisés en "cermet", c'est-à-dire en un matériau composite à matrice métallique comprenant un renfort en céramique, par exemple de type alumine/platine, la partie principale du corps, isolante, étant en céramique (ici en alumine) et les éléments conducteurs 126 étant en platine. En variante, on peut utiliser un composite de type alumine/tungstènemolybdène. On peut également utiliser une céramique de type carbure de silicium.

Les procédés de fabrication de tels éléments, qui présentent l'avantage d'une transition continue entre la partie isolante et la partie conductrice, sont largement maitrisés et permettent de fabriquer un module avec un dimensionnement adapté à la présente application (on reviendra dans la suite sur le dimensionnement des différentes parties du dispositif).

Par ailleurs, le module 120 réalisé de cette manière permet, en coopération avec un capot 150 tel qu'on le décrira en détail dans la suite, de garantir une herméticité absolue de la cavité abritant le circuit intégré 130, ce qui pourrait être délicat à obtenir avec un simple surmoulage d'une matière synthétique injectée sur des conducteurs métalliques.

En référence aux Figures 8 et 9, on a illustré la façon dont le capot 150 de protection du circuit 130 est constitué et fixé de façon hermétique sur la face du module 120 qui porte le circuit intégré 130.

Ce capot est également réalisé ici en technologie cermet et comprend une partie principale isolante formant une cavité 154 destinée à abriter le circuit 30 et une zone annulaire conductrice 152 tournée vers le module 120. En association avec ce capot, il est prévu sur le module 120 une zone conductrice supplémentaire 128 de forme générale annulaire, par exemple de même géométrie que la zone annulaire 152 du capot, affleurant à la surface qui porte le circuit 130 en étant agencée autour de ce dernier. On notera que la technologie cermet de fabrication du module 120 permet facilement d'un intégrer une telle zone conductrice annulaire.

Avec une telle configuration, une fois connectés les fils de *bonding* 140, le capot 150 est appliqué et maintenu contre la face du module 120 portant le circuit 130 et une soudure par tirs laser point par point est alors mise en oeuvre en une pluralité d'endroits à la jonction entre les zones annulaires 128, 152, les points de soudure étant désignés sur la Figure 9 par la référence 170. Ce processus permet d'assurer une liaison entièrement hermétique entre le module 120 et le capot 150, pour ainsi parfaitement protéger le circuit 130 et ses liaisons.

On notera ici que ce principe de fermeture de la cavité abritant le circuit 130 permet de ne pas provoquer une élévation trop importante de la température au sein de la cavité, celle-ci pouvant être maintenue en deçà de 400 °C.

On observera également ici que la configuration des Figures 2 et 3 est légèrement différente de celle des figures 5 à 9. Sur les Figures 2 et 4, la zone conductrice destinée au soudage hermétique du capot 150 est désignée par la référence 129. Elle s'étend non pas dans un plan général radial mais selon un cylindre périphérique au niveau d'une zone de diamètre réduit, adjacente à sa face supportant le circuit 130, du module 120. Enfin selon une variante, le capot soudé 150 peut être réalisé entièrement en métal biocompatible, par exemple en alliage de titane.

Selon une autre variante encore, le capot peut être fixé hermétiquement sur l'élément 120 par collage.

Dans tous les cas, il est avantageux de renforcer encore la protection du circuit et de ses connexions associées en encapsulant l'ensemble formé par l'élément 120 et son capot 150, abritant le circuit 130, et les fils connectés, dans un bloc de polymère souple 160, par exemple de silicone, ce bloc emprisonnant également une courte longueur des fils 202, 302 pour sécuriser mécaniquement l'ensemble.

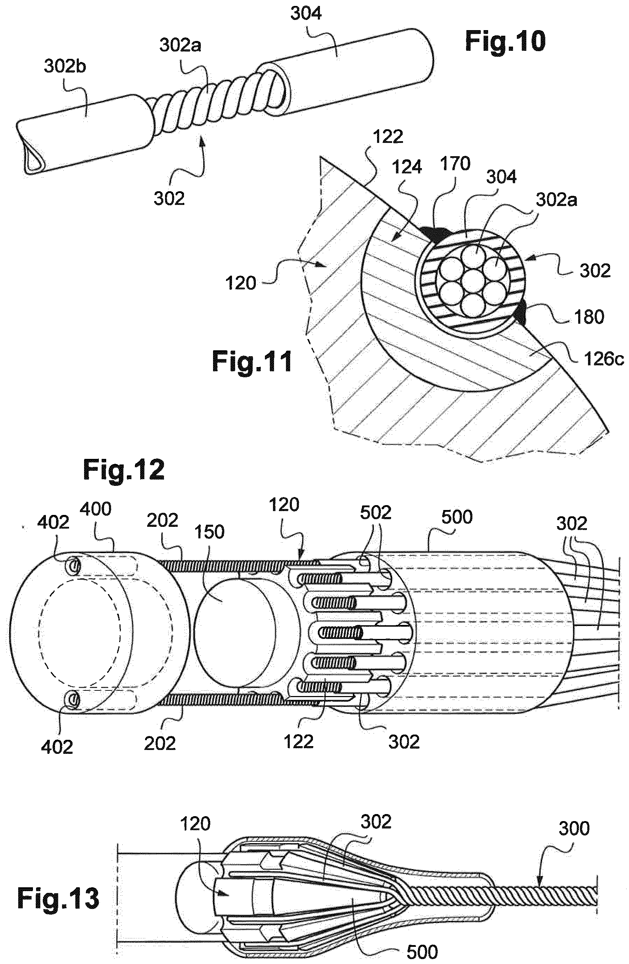

En référence maintenant aux Figures 10 et 11, on va décrire en détail la manière dont les conducteurs 202, 302 sont maintenus mécaniquement et connectés électriquement au module 120, au niveau des gorges respectives 124. Avantageusement et comme illustré sur la Figure 10, un tel conducteur, ici un conducteur 302 côté électrodes, reçoit au niveau d'une partie d'extrémité 302a dénudée de sa gaine isolante 302b, un hypotube 304 destiné à faciliter le processus de connexion. Cet hypotube, ici en platine, peut être soudé au conducteur 302 par tir laser, ou simplement enfilé sur celui-ci au montage.

Comme le montre la Figure 11, l'extrémité du conducteur pourvu de son hypotube 304 est positionnée et maintenue au droit d'une gorge 124 correspondant à sa destination finale. On observe que la partie conductrice 126c débouchant dans la gorge 124 forme une cavité semi-cylindrique dont le diamètre est voisin du diamètre extérieur de l'hypotube 304, de manière que ce dernier s'y loge intimement.

On réalise ensuite un tir laser au niveau de la transition entre l'hypotube 304 et la partie conductrice 126c, de chaque côté (ou plusieurs tirs de chaque côté), pour assurer la fixation mécanique et la connexion électrique de l'ensemble formé de l'âme 302a du conducteur et de l'hypotube 304 avec la partie conductrice 126c de l'élément conducteur 126, lui-même connecté à l'extrémité opposée au circuit intégré 130.

On notera ici que l'hypotube 304 permet de limiter les risques de mauvaise connexion lors du tir laser. Il peut être omis dans le cas où la fiabilité du processus de tir laser est suffisante, auquel cas l'âme conductrice 302a du conducteur 302 est directement soudée sur la partie 126c de l'élément conducteur 126 (et de même pour les conducteurs 202).

Selon une variante de réalisation non illustrée, la liaison électrique entre le module 120 et les conducteurs 202, 302 peut être réalisée sans recourir à une soudure par tirs laser. Plus précisément, en plaçant les conducteurs 202, 302 dans leur gorge respective 124 et en appliquant autour de l'ensemble un cerclage, par exemple à l'aide d'une bague en PEEK (polyéther-éther-cétone), qui vient sertir les conducteurs 202, 302 contre leurs parties conductrices respectives 126c. Une légère conicité de la périphérie 122 du module peut être prévue pour assurer cette fonction, la bague étant déplacée vers la partie de plus grand diamètre extérieur du module, puis collée.

En référence maintenant à la Figure 12, on prévoit avantageusement d'associer au module 120 porte-circuit et de connexion des pièces de transition, respectivement 400, 500, destinées à assurer un prépositionnement des connecteurs 202, 302 à relier au module, respectivement.

Ainsi la pièce 400, réalisée en matière synthétique injectée, présente la forme d'un cylindre d'un diamètre extérieur voisin de celui du module 120, et présente dans des régions diamétralement opposées deux orifices traversants 402 généralement parallèles dans la direction axiale, la distance entre ces orifices étant voisine de la distance entre deux gorges diamétralement opposées 124 du module. Les deux conducteurs 202 sont enfilés dans ces deux orifices puis engagés dans leur gorge respective 124, la pièce 400 assurant un prépositionnement des deux conducteurs pendant les opérations de soudage ou de sertissage.

Dans le même esprit, une pièce 500 également en matière synthétique injectée comporte ici dix orifices traversants 502 généralement parallèles dans la direction axiale, dans lesquels sont enfilés les dix conducteurs 302 avant leur mise en place dans leur gorge respective. Cette pièce 500 de transition est, ici, généralement cylindrique.

On comprend que ces pièces de transition peuvent être particulièrement utiles, en particulier du côté de la partie principale 300 de la sonde, lorsqu'un très grand nombre de conducteurs doit être positionné sur le module 120.

Après soudage ou sertissage des conducteurs, ces pièces 400, 500, par exemple collées de part et d'autre du module 120 doté de son capot 150, assurent une stabilité dimensionnelle de l'ensemble et évitent que les conducteurs soient accidentellement pliés et éventuellement sectionnés lors des manipulations.

Comme illustré sur la Figure 13, on peut donner à la pièce de transition 500 une forme généralement conique, les orifices 502 convergeant à partir d'une zone adjacente au module 120, où ils adoptent une disposition correspondant à celle des gorges homologues, en direction d'une zone distale resserrée où l'ensemble des conducteurs 302 se rejoint dans la partie de sonde 300.

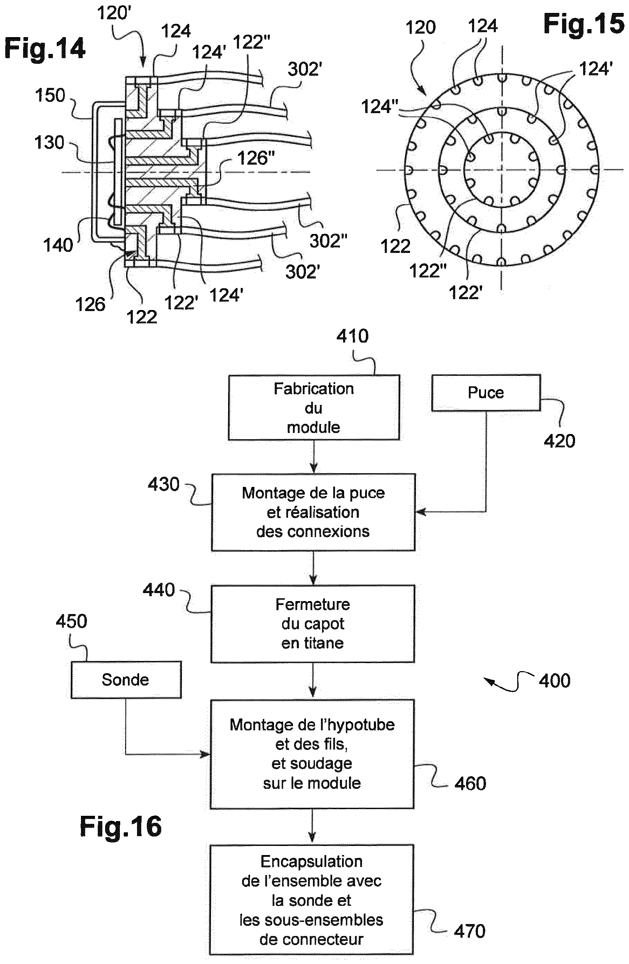

On va maintenant décrire en référence aux Figures 14 et 15 une variante de réalisation module porte-circuit et de connexion, permettant la connexion d'un nombre accru de conducteurs 302.

Dans cette variante, le module porte-circuit et de connexion, désigné par la référence 120', comprend trois étages cylindriques de connexions périphériques avec les conducteurs, coaxiaux et de diamètres progressivement décroissants à mesure que l'on s'éloigne de la partie supportant le circuit intégré 130.

Ainsi les Figures 14 et 15 illustrent une première série de gorges 124 pratiquées au niveau de l'étage le plus large, une deuxième série de gorges 124' pratiquées au niveau de l'étage suivant, de diamètre intermédiaire, et une troisième série de gorges 124" pratiquées au niveau du dernier étage, de plus petit diamètre.

Les périphéries cylindriques des trois étages sont désignées par les références 122, 122' et 122".

Le module 120 abrite en son sein trois groupes d'éléments conducteurs noyés, respectivement 126, 126' et 126", dont les configurations sont adaptées à la géométrie du corps du module pour offrir chacun une surface de connexion affleurante, ces surfaces de connexion étant réparties autour du circuit.

On comprend qu'une telle configuration permet d'accroitre considérablement la densité de connexion. Il devient possible de connecter typiquement jusqu'à une centaine de conducteurs 302 ou davantage, pour ainsi réaliser des sondes pourvues de très nombreuses électrodes, offrant d'excellentes possibilités de localisation de la stimulation.

Avantageusement, le module 120' selon cette variante est également fabriqué par la technologie Cermet, et la pièce de transition 500, si une telle pièce est prévue, est adaptée en conséquence.

L'invention permet de réaliser une sonde multiélectrode avec microconducteurs, d'un diamètre typique de 0,3 mm avec les technologies actuelles, avec une partie intermédiaire dédiée à la fois à la connexion avec un connecteur (par exemple un connecteur standard de type *IS-1* ou *IS-4*) et au démultiplexage, dont le diamètre n'excède pas 3 à 4 mm (avec une puce d'une taille de 1 mm² qui peut être réalisée avec les performances d'intégration actuelles).

La présente invention présente de nombreux avantages, parmi lesquels les avantages suivants :
- elle rend possible l'intégration du démultiplexage dans la sonde, en conservant pour celle-ci un petit diamètre, typiquement de l'ordre de 0,3 mm dans les technologies actuelles, avec une transition progressive entre le logement pour le circuit de démultiplexage et la sonde elle-même ;
- elle est compatible avec un connecteur standard (par exemple connecteur 2 fils de type *IS-1* ou connecteur 4 fils de type *IS-4*) et permet de miniaturiser la région dédiée au démultiplexage des signaux arrivant sur ces connecteurs ;
- la fabrication peut être économique, avec des soudures par robot de tir laser sur des composants dont le prix de revient peut demeurer raisonnable ;
- elle permet d'augmenter considérablement le nombre de connexions (100 connexions, voire davantage) tout en conservant une taille raisonnable pour la partie de démultiplexage ;
- elle permet d'assurer une herméticité et une protection parfaites de la région abritant le circuit intégré de démultiplexage, grâce à la fixation hermétique du capot et le cas échéant l'encapsulation dans un polymère souple ;
- elle permet des liaisons électriques très courtes, avec par conséquent un faible risque de connexions incorrectes du fait de ruptures de conducteurs.

En ce qui concerne plus particulièrement le processus de fabrication d'une sonde multiélectrode réalisée selon les enseignements de la présente invention, celui-ci peut se résumer aux étapes présentées schématiquement sur l'organigramme 400 de la Figure 16, à savoir :
- fabrication du module 120 (étape 410) ;
- parallèlement, préparation de la puce de circuit intégré 130 (étape 420) ;
- montage de la puce 130 sur le module 120 et réalisation des *bondings* 140 de connexion (étape 430) ;
- pose et scellement du capot en titane 150 sur le module 120 (étape 440) ;
- préparation de la sonde (étape 450), puis montage de l'hypotube 304 et mise en place et soudage des fils 202, 302 sur le module (étape 460);
- enfin, encapsulation de l'ensemble avec la sonde et les divers sous-ensembles du connecteur 110 (étape 470).

## Revendications

1. Sonde multiélectrode (300, 100) pour stimulation localisée, comprenant :
- un connecteur (110) pour une liaison de commande et/ou d'alimentation et/ou de transfert de données (200) ;
- un circuit intégré de démultiplexage (130) apte à recevoir en entrée sur des premiers conducteurs (202) des signaux électriques de commande et/ou d'alimentation et/ou de transfert de données en provenance de la liaison de commande et/ou d'alimentation et/ou de transfert de données (200) et relié en sortie à une pluralité de seconds conducteurs (302) contenus dans la sonde ;
- un élément porte-circuit et de connexion (120) possédant un corps formant support pour le circuit intégré (130) de démultiplexage et logé dans un corps de connecteur (100) portant le connecteur (110) ; et
- un ensemble d'électrodes (306) s'étendant le long de la sonde et reliées aux seconds conducteurs; sonde dans laquelle l'élément porte-circuit et de connexion (120) :
- est de forme généralement cylindrique et reçoit le circuit intégré (130) sur l'une de ses faces d'extrémité ;
- définit un ensemble de cavités de connexion (124) présentant la forme de gorges s'étendant axialement à la périphérie (122) du corps de l'élément (120), avec au moins les seconds conducteurs (302) répartis à la périphérie (122) du corps autour d'un axe général dudit corps et disposés dans lesdites cavités de connexion (124) ; et
- comprend un ensemble d'éléments de connexion (126) noyés dans le matériau du corps, émergeant au niveau d'une région de l'élément supportant le circuit intégré et au niveau de cavités respectives, ces éléments de connexion reliant électriquement lesdits premiers et/ou seconds conducteurs (202, 302) à des bornes respectives dudit circuit intégré (130).

2. La sonde de la revendication 1, dans laquelle les éléments conducteurs (126) possèdent chacun une partie émergente (126c) s'étendant dans une gorge respective sur au moins une partie de sa longueur axiale.

3. La sonde de la revendication 1, dans laquelle les éléments conducteurs (126) possèdent chacun une première partie noyée (126a) s'étendant généralement axialement et dont une extrémité débouche au niveau d'une surface adjacente au circuit intégré (130).

4. La sonde de la revendication 2, dans laquelle chaque élément conducteur (126) comprend une partie intermédiaire noyée (126b) s'étendant généralement radialement entre la première partie noyée (126a) et la partie émergente (126c) s'étendant dans la gorge.

5. La sonde de la revendication 1, dans laquelle le circuit intégré (130) est protégé par un capot (150) fixé de façon hermétique sur le corps de l'élément porte-circuit et de connexion (120).

6. La sonde de la revendication 1, dans laquelle l'élément porte-circuit et de connexion (120) est réalisé par une technologie céramique-métal, le corps de l'élément étant constitué par une région en céramique et les éléments de connexion (126) étant constituées par des régions en métal.

7. La sonde des revendications 5 et 6 prises en combinaison, dans laquelle l'élément porte-circuit et de connexion (120) comporte en outre une région métallique (128 ; 129) pour le soudage du capot (150).

8. La sonde de la revendication 7, dans laquelle la région métallique (128 ; 129) est une région annulaire entourant le circuit intégré (130).

9. La sonde de la revendication 7, dans laquelle le capot (150) est également réalisé en technologie céramique-métal et comprend une région métallique (152) homologue de la région métallique (128 ; 129) entourant le circuit intégré (130).

10. La sonde de la revendication 6, dans laquelle les conducteurs (202, 302) sont entourés à leur extrémité libre reçue dans sa cavité respective (124) par un manchon métallique (304).

11. La sonde de la revendication 1, comprenant en outre un élément de transition (500) apte à retenir les seconds conducteurs (302) dans une configuration correspondant à l'agencement des cavités (124) pour lesdits seconds conducteurs.

12. La sonde de la revendication 1, comprenant en outre un élément de transition (400) apte à retenir les premiers conducteurs (202) dans une configuration correspondant à l'agencement des cavités (124) pour lesdits premiers conducteurs.

13. La sonde de la revendication 1, dans laquelle le corps de l'élément porte-circuit et de connexion (120) comprend au moins deux régions cylindriques (122, 122', 122") généralement coaxiales et de diamètres différents, chaque région possédant une pluralité de gorges (124, 124', 124") s'étendant axialement à sa périphérie.

14. Procédé de connexion de conducteurs (202, 302) à un circuit de démultiplexage (130) dans une sonde multiélectrode (300, 100) de stimulation localisée, ladite sonde comprenant :
- un circuit de démultiplexage apte à recevoir en entrée sur des premiers conducteurs (202) des signaux électriques de commande et/ou d'alimentation et/ou de transfert de données et relié en sortie à une pluralité de seconds conducteurs (302) ;
- un ensemble d'électrodes (306) s'étendant le long de la sonde et reliées aux seconds conducteurs ;
- un élément porte-circuit et de connexion (120) de forme généralement cylindrique réalisé en technologie céramique-métal et possédant un corps céramique formant support pour un circuit intégré de démultiplexage (130) et définissant un ensemble de cavités (124) de connexion présentant la forme de gorges s'étendant axialement à la périphérie (122) du corps avec au moins les seconds conducteurs (302), réparties à la périphérie (122) du corps autour d'un axe général dudit corps ; et
- un ensemble de régions de connexion métalliques (126) noyées dans le corps et émergeant au voisinage d'une région de l'élément supportant le circuit (130) et au niveau desdites cavités (124),
ce procédé étant **caractérisé par** des étapes consistant à :
a) monter le circuit intégré de démultiplexage (130) sur l'une des faces d'extrémité de l'élément (120) ;
b) relier des bornes (132) du circuit aux parties émergentes (126a) des régions de connexion (126) voisines du circuit ; et
c) relier les conducteurs (302) aux parties émergentes (126c) des régions de connexion (126) au niveau desdites cavités.

15. Le procédé de la revendication 14, dans lequel l'étape b) est mise en oeuvre à l'aide de fils de liaison (140).

16. Le procédé de la revendication 14, dans lequel les étapes a) et b) sont mises en oeuvre simultanément par une technique de montage de puce retournée.

17. Le procédé de la revendication 14, dans lequel l'étape c) est réalisée par tirs laser.

18. Le procédé de la revendication 17, dans lequel les conducteurs (302) sont entourés à leur extrémité libre reçue dans sa cavité respective (124) par un manchon métallique (304).

19. Le procédé de la revendication 18, dans lequel le matériau métallique du manchon est le même que le matériau métallique de l'élément porte-circuit et de connexion (120).

20. Le procédé de la revendication 14, comprenant également des étapes consistant à :
d) prévoir un capot (150) au moins en partie métallique pour la protection du circuit intégré (130) ;
e) prévoir sur l'élément porte-circuit et de connexion (120) une région métallique (128 ; 129) entourant le circuit intégré ; et
f) fixer le capot sur ledit élément par tirs laser au niveau de ladite région métallique.

## Patentansprüche

1. Multielektrodensonde (300, 100) zur örtlichen Stimulation, umfassend:
- einen Verbinder (110) für eine Steuerungs- und/oder Stromversorgungs- und/oder Datenübertragungsverbindung (200);
- einen integrierten Schaltkreis zur Demultiplexierung (130), der dazu geeignet ist, an ersten Leitern (202) als Eingabe elektrische Steuerungs- und/oder Stromversorgungs- und/oder Datenübertragungssignale von der Steuerungs-und/oder Stromversorgungs- und/oder Datenübertragungsverbindung (200) zu empfangen und am Ausgang mit einer Vielzahl von zweiten in der Sonde enthaltenen Leitern (302) verbunden ist;
- ein Schaltungsträger- und Verbindungselement (120), das einen Körper aufweist, der als Träger für den integrierten Schaltkreis zur Demultiplexierung (130) dient und in einem den Verbinder (110) tragenden Verbinderkörper (100) aufgenommen ist; und
- einen sich entlang der Sonde erstreckenden und mit den zweiten Leitern verbundenen Satz von Elektroden (306);
wobei in der Sonde, das Schaltungsträger- und Verbindungselement (120):
- eine allgemein zylindrische Form aufweist und den integrierten Schaltkreis (130) auf einer seiner Endflächen aufnimmt;
- einen Satz von nutförmigen Verbindungsausnehmungen (124) bestimmt, die sich am Umfang (122) des Körpers des Elements (120) axial erstrecken, wobei wenigstens die zweiten Leiter (302) am Umfang (122) des Körpers um eine allgemeine Achse des Körpers verteilt sind und in den Verbindungsausnehmungen (124) angeordnet sind; und
- einen Satz von in dem Material des Körpers eingebetteten Verbindungselementen (126) umfasst, die in einem Abschnitt des den Schaltkreis tragenden Elements und in jeweiligen Ausnehmungen hervortreten, wobei diese Verbindungselemente die ersten und/oder zweiten Leiter (202, 302) mit jeweiligen Klemmen des integrierten Schaltkreises (130) elektrisch verbinden.

2. Sonde nach Anspruch 1, bei der die Leitelemente (126) jeweils einen hervorstehenden Teil (126c) aufweisen, der sich in einer jeweiligen Nut über mindestens einen Teil ihrer axialen Länge erstreckt.

3. Sonde nach Anspruch 1, bei der die Leitelemente (126) jeweils einen ersten eingebetteten Teil (126a) aufweisen, der sich im Allgemeinen axial erstreckt und an einem Ende an einer an den Schaltkreis (130) angrenzenden Fläche hervortritt.

4. Sonde nach Anspruch 2, bei der jedes Leitelement (126) einen eingebetteten Mittelteil (126b) aufweist, der sich zwischen dem ersten eingebetteten Teil (126a) und dem sich in der Nut erstreckenden hervorstehenden Teil (126c) im Allgemeinen radial erstreckt.

5. Sonde nach Anspruch 1, bei der der Schaltkreis (130) durch einen Deckel (150) geschützt ist, der am Körper des Schaltungsträger- und Verbindungselements (120) hermetisch befestigt ist.

6. Sonde nach Anspruch 1, bei der das Schaltungsträger- und Verbindungselement (120) mittels einer Keramik-Metall-Technik hergestellt ist, wobei der Körper des Elements aus einem keramischen Abschnitt und die Verbindungselemente (126) aus metallischen Abschnitten bestehen.

7. Sonde nach den Ansprüchen 5 und 6 in Kombination miteinander, bei der das Schaltungsträger- und Verbindungselement (120) außerdem einen metallischen Abschnitt (128; 129) zum Anschweißen des Deckels (150) umfasst.

8. Sonde nach Anspruch 7, bei der der metallische Abschnitt (128; 129) ein ringförmiger Abschnitt ist, der den integrierten Schaltkreis (130) umgibt.

9. Sonde nach Anspruch 7, bei der der Deckel (150) ebenfalls mittels einer Keramik-Metall-Technik hergestellt ist und einen metallischen Abschnitt (152) als Gegenstück zu dem den integrierten Schaltkreis (130) umgebenden metallischen Abschnitt (128; 129) aufweist.

10. Sonde nach Anspruch 6, bei der die Leiter (202, 302) an ihrem in seiner jeweiligen Ausnehmung (124) aufgenommenen freien Ende durch eine Metallhülse (304) umgeben sind.

11. Sonde nach Anspruch 1, weiter umfassend ein Übergangselement (500), das dazu geeignet ist, die zweiten Leiter (302) in einer Konfiguration zu halten, die der Anordnung der Ausnehmungen (124) für die zweiten Leiter entspricht.

12. Sonde nach Anspruch 1, weiter umfassend ein Übergangselement (400), das dazu geeignet ist, die ersten Leiter (202) in einer Konfiguration zu halten, die der Anordnung der Ausnehmungen (124) für die ersten Leiter entspricht.

13. Sonde nach Anspruch 1, bei der der Körper des Schaltungsträger- und Verbindungselementes (120) mindestens zwei im Allgemeinden koaxiale zylindrische Abschnitte (122, 122', 122") mit verschiedenen Durchmessern umfasst, wobei jeder Abschnitt eine Vielzahl von Nuten (124, 124', 124") aufweist, die sich an seinem Umfang axial erstrecken.

14. Verfahren zur Verbindung von Leitern (202, 302) an einen Schaltkreis zur Demultiplexierung (130) in einer Multielektrodensonde (300, 100) zur örtlichen Stimulation, wobei die Sonde Folgendes umfasst:
- einen Schaltkreis zur Demultiplexierung, der dazu geeignet ist, an ersten Leitern (202) als Eingabe elektrische Steuerungs- und/oder Stromversorgungs- und/oder Datenübertragungssignale zu empfangen und am Ausgang mit einer Vielzahl von zweiten Leitern (302) verbunden ist;
- einen sich entlang der Sonde erstreckenden und mit den zweiten Leitern verbundenen Satz von Elektroden (306);
- ein im Allgemeinen zylindrisches Schaltungsträger- und Verbindungselement (120), das mittels einer Keramik-Metall-Technik hergestellt ist und einen keramischen Körper aufweist, der als Träger für einen integrierten Schaltkreis zur Demultiplexierung (130) dient und einen Satz von nutförmigen Verbindungsausnehmungen (124) bestimmt, die sich am Umfang (122) des Körpers axial erstrecken, und wobei wenigstens die zweiten Leiter (302) am Umfang (122) des Körpers um eine allgemeine Achse des Körpers verteilt sind; und
- eine Vielzahl von metallischen Verbindungsabschnitten (126), die im Körper eingebettet sind und in der Nähe eines Abschnitts des den Schaltkreis (130) tragenden Elementes und in der Nähe der Ausnehmungen hervortreten,
wobei das Verfahren durch folgende Schritte gekennzeichnet ist:
a) Montieren des integrierten Schaltkreises zur Demultiplexierung (130) auf einer der Endflächen des Elementes (120);
b) Verbinden der Klemmen (132) des Schaltkreises mit den hervorstehenden Teilen (126a) der in der Nähe des Schaltkreises befindlichen Verbindungsabschnitten (126); und
c) Verbinden der Leiter (302) mit den hervorstehenden Teilen (126c) der Verbindungsabschnitte (126) an den Ausnehmungen.

15. Verfahren nach Anspruch 14, bei dem der Schritt b) mit Hilfe von Verbindungdrähten umgesetzt wird.

16. Verfahren nach Anspruch 14, bei dem die Schritte a) und b) simultan durch eine Flip-Chip-Montagetechnik umgesetzt werden.

17. Verfahren nach Anspruch 14, bei dem der Schritt c) durch Laserschüsse umgesetzt wird.

18. Verfahren nach Anspruch 17, bei dem die Leiter (302) an ihrem in seiner jeweiligen Ausnehmung (124) aufgenommenen freien Ende durch eine Metallhülse (304) umgeben sind.

19. Verfahren nach Anspruch 18, bei dem das metallische Material der Hülse dasselbe wie das metallische Material des Schaltungsträger- und Verbindungselementes ist.

20. Verfahren nach Anspruch 14, das ebenfalls folgende Schritte umfasst:
d) einen zumindest teilweise metallischen Deckel (150) zum Schutz des integrierten Schaltkreises (130) vorsehen;
e) auf dem Schaltungsträger- und Verbindungselement (120) einen den integrierten Schaltkreis umgebenden metallischen Abschnitt (128, 129) vorsehen; und
f) den Deckel durch Laserschüsse an dem metallischen Abschnitt am Element befestigen.

## Claims

1. A multi-electrode probe (300, 100) for localized stimulation, comprising:
- a connector (110) for an electrical control and/or power supply and/or data transfer connection (200);
- an integrated demultiplexing circuit (130) adapted to receive as an input, on a plurality of first conductors (202), electrical control and/or power supply and/or data transfer signals from the electrical control and/or power supply and/or data transfer connection (200), and connected as an output to a plurality of second conductors (302) contained in the probe;
- a circuit carrier and connection element (120) having a body forming a support for the demultiplexing integrated circuit (130) and housed in a connector body (100) carrying the connector (110) ; and
- an electrode assembly (306) extending along the probe and connected to the second conductors;
wherein the circuit carrier and connection element (120):
- is of a generally cylindrical shape and receives the integrated circuit (130) on one of its end faces;
- defines a set of connection cavities (124) having the shape of grooves extending axially in a periphery (122) of the body of the element (120), with at least the second conductors (302) distributed around the periphery (122) of the body around a general axis of the body and disposed in the connection cavities (124); and
- comprises a set of connection elements (126) embedded in the material of the body, emerging at a region of the element supporting the integrated circuit and at respective cavities, these connection elements electrically connecting the first and/or second conductors (202, 302) to respective terminals of the integrated circuit (130).

2. The probe according to claim 1, wherein the conductive elements (126) each have a protruding part (126c) extending in a respective groove on at least part of its axial length.

3. The probe according to claim 1, wherein the conductive elements (126) each have a first embedded portion (126a) extending generally axially and having one end emerging at a surface adjacent to the integrated circuit (130).

4. The probe according to claim 2, wherein each conductive element (126) comprises an intermediate embedded portion (126b) extending generally radially between the first embedded portion (126a) and the emerging part (126c) extending in the groove.

5. The probe according to claim 1, wherein the integrated circuit (130) is protected by a cover (150) sealingly fixed to the body of the circuit carrier and connection element (120).

6. The probe according to claim 1, wherein the circuit carrier and connection element (120) is formed by a metal-ceramic technology, the body of the element comprising a ceramic region and the connection elements (126) comprising metal regions.

7. The probe according to claims 5 and 6 combined, wherein the circuit carrier and connection element (120) further comprises a metal region (128; 129) for welding the cover (150).

8. The probe according to claim 7, wherein the metal region (128; 129) is an annular region surrounding the integrated circuit (130).

9. The probe according to claim 7, wherein the cover (150) is made of metal-ceramic technology and comprises a metal region (152) that is the counterpart of the metal region (128; 129) surrounding the integrated circuit (130).

10. The probe according to claim 6, wherein the conductors (202, 302) are surrounded at a free end received in a respective cavity (124) by a metal sleeve (304).

11. The probe according to claim 1, further comprising a transition member (500) adapted to retain the second conductors (302) in a configuration corresponding to the arrangement of the cavities (124) for the second conductors.

12. The probe according to claim 1, further comprising a transition member (400) adapted to retain the first conductors (202) in a configuration corresponding to the arrangement of the cavities (124) for the first conductors.

13. The probe according to claim 1, wherein the body of the circuit carrier and connection element (120) comprises at least two cylindrical regions (122, 122', 122"), generally coaxial and of different diameters, each region having a plurality of grooves (124, 124', 124") axially extending at a periphery.

14. A method for connection of conductors (202, 302) to a demultiplexing circuit (130) in a multi-electrode probe (300, 100) for localized stimulation, the probe comprising:
- a demultiplexing circuit capable of receiving at its input on first conductors (202) electrical control and/or power supply and/or data transfer signals and connected at its output to a plurality of second conductors (302);
- an electrode assembly (306) extending along the probe and connected to the second conductors;
- a circuit carrier and connection element (120) having a generally cylindrical shape and formed by a metal-ceramic technology and having a ceramic body forming a support for a demultiplexing integrated circuit (130) and defining a set of connection cavities (124) having the shape of grooves extending axially in a periphery (122) of the body with at least the second conductors (302) distributed around the periphery (122) of the body around a general axis of the body;and
- a set of metal connection regions (126) embedded in the body and emerging at a region of the element supporting the circuit (130) and at the cavities (124),
the method being **characterized by** the following steps:
a) mounting the demultiplexing integrated circuit (130) on one of the end faces of the element (120);
b) connecting terminals (132) of the circuit to the emerging portions (126a) of the connection regions (126) adjacent to the circuit; and
c) connecting the conductors (302) to the emerging portions (126c) of the connection regions (126) at the cavities.

15. The method according to claim 14, wherein step b) is done using connection wires (140).

16. The method according to claim 14, wherein steps a) and b) are implemented simultaneously by an inverted chip mounting technique.

17. The method according to claim 14, wherein step c) is performed by laser shots.

18. The method according to claim 17, wherein the conductors (302) are surrounded at their free end received in its respective cavity (124) by a metal sleeve (304).

19. The method according to claim 18, wherein the metallic material of the sleeve is the same as a metallic material of the circuit carrier and connection element (120).

20. The method according to claim 14, further comprising the steps of:
d) providing a cover (150) at least partially made of metal to protect the integrated circuit (130);
e) providing on the circuit carrier and connection element (120) a metal region (128; 129) surrounding the integrated circuit; and
f) fixing the cover to said element by laser shots at said metal region.
